# EUROPEAN PATENT APPLICATION

(11) **EP 3 473 110 A1**
(43) Date of publication of application: **24.04.2019**
(21) Application number: 18200979.5
(22) Date of filing: 17.10.2018
(51) Int. Cl.: A23L 27/30, A23L 33/105, A23L 33/125

(54) **SWEETENER COMPOSITION AND CONSUMABLE COMPRISING A SWEETENER COMPOSITION**

(30) Priority: 19.10.2017 US 201715787939
(71) Applicant: Zucozero GmbH, 10119 Berlin (DE)
(72) Inventor: Watson, Miles Alexander, 10437 Berlin (DE)
(74) Representative: Patentanwälte Ruff, Wilhelm, Beier, Dauster & Partner mbB

(57) **Abstract**

The invention relates to a sweetener composition comprising
- at least one sugar alcohol,
- at least one reducing monosaccharide and/or at least one reducing disaccharide,
- at least one oligosaccharide and/or at least one polysaccharide,
- at least one amino acid,
- at least one anti-laxative agent,
- at least one biocompatible alkali metal salt and/or at least one biocompatible alkaline earth metal salt and
- monk fruit and/or a product of monk fruit and/or at least one sweetener of monk fruit.

Further, the invention refers to a consumable, in particular edible consumable, comprising the sweetener composition.

## Description

### FIELD OF THE INVENTION AND TECHNICAL BACKGROUND OF THE INVENTION

The present invention relates to a sweetener composition and to a consumable, in particular edible consumable, comprising a sweetener composition.

Generally, sweetener compositions are known.

For example, a sweetener composition comprising a sweetener and deoxycholic acid or a derivative or a stereoisomer or a salt or a hydrate thereof is known from EP 2 606 747 A1.

WO 2012/107206 A1 refers to a sweetener composition comprising a sweetener and microcarpalide or a derivative or a stereoisomer or a salt or a hydrate thereof.

US 2014/0199246 A1 discloses a sweet ingestible composition comprising rebaudioside B and at least one sweetener selected from the group consisting of steviol glycosides, stevia extracts, natural sweeteners, glycosylated terpenoid sweeteners, synthetic high intensity sweeteners, oligosaccharides, caloric sweeteners, and combinations thereof.

A natural sweetener composition comprising a sugar alcohol, a soluble fibre blend, a rheology modifier, and an enhancing natural flavour is known from US 2015/0208703 A1.

A serious withdrawal of conventional sweetener compositions is their disability of caramelizing when being heated up. Instead, these compositions often just burn during baking or cooking.

### PROBLEM AND SOLUTION

Therefore, the problem underlying the present invention is to provide a sweetener composition, in particular for sugar substitute, that is able to caramelize while baking or cooking.

This problem is properly addressed by a sweetener composition as defined in independent claims 1 and 28 and by a consumable as defined in claim 27. Preferred embodiments are defined in the dependent claims and the description. The wording of all claims is incorporated herein by specific reference.

According to a first aspect the invention relates to a sweetener composition, preferably for sugar substitute, in particular for sucrose (table sugar) substitute and/or for glucose substitute.

The sweetener composition comprises or includes the following ingredients:
- at least one sugar alcohol,
- at least one reducing monosaccharide and/or at least one reducing disaccharide,
- at least one oligosaccharide and/or at least one polysaccharide,
- at least one amino acid,
- at least one anti-laxative agent,
- at least one biocompatible alkali metal salt and/or at least one biocompatible alkaline earth metal salt and
- monk fruit and/or a product of monk fruit and/or at least one sweetener of monk fruit.

The term "at least one" as used according to the present invention may define one type of the respective ingredient of the sweetener composition, or a combination or mixture of different types, i.e. two or more different types, of the respective ingredient of the sweetener composition. For example, the term "at least one sugar alcohol" may mean one type of sugar alcohol or a combination or mixture of different sugar alcohols, i.e. two or more different sugar alcohols.

The term "reducing monosaccharide" as used according to the present invention refers to a monosaccharide that is capable of acting as a reducing agent, i.e. that is capable of reducing another compound, typically because it has a free hemiacetal group or because it is convertible to an open-chain form with a free aldehyde group or an open-chain form with a free ketone group, wherein the conversion to the open-chain form preferably takes place in water or an aqueous environment, in particular in aqueous solution. Monosaccharides which can be converted to open-chain form with an aldehyde group are known as aldoses, and those which can be converted to open-chain form with a ketone group are known as ketoses. Ketoses must first tautomerize to aldoses before they can act as a reducing agent. Therefore, a ketone-bearing monosaccharide like fructose is considered as a reducing monosaccharide but it is the isomer containing the aldehyde group which is reducing since ketones cannot be oxidized without decomposition of the monosaccharide.

The term "reducing disaccharide" as used according to the present invention refers to a disaccharide that is capable of acting as a reducing agent, i.e. that is capable of reducing another compound, typically because it has a free hemiacetal group or because it is convertible to an open-chain form with a free aldehyde group or an open-chain form with a free ketone group, wherein the conversion to the open-chain form preferably takes place in water or an aqueous environment, in particular in aqueous solution.

The term "oligosaccharide" as used according to the present invention defines a saccharide polymer containing or consisting of 2 to 10 monosaccharide units.

The term "polysaccharide" as used according to the present invention defines a saccharide polymer containing or consisting of more than 10 monosaccharide units, in particular 11 to 100 monosaccharide units.

The term "anti-laxative agent" as used according to the present invention defines an agent which is able to reduce or eliminate a (possible) laxative effect of the at least one sugar alcohol.

The term "monk fruit" as used according to the present invention means the fruits of herbaceous perennial vine of the Cucurbitaceae. Monk fruit may be also denoted as luo han gou fruit. The monk fruit is notable for its sweetness, which can be concentrated from its juice. The sweet taste of monk fruit comes mainly from mogrosides, a group of triterpene glycosides. Through solvent extraction, a powder containing 80 % mogrosides can be obtained, the main one being mogrosides V. Other similar agents in the fruit are siamenoside and neomogroside. For further details of the monk fruit reference is made to the textbook "Sweeteners and Sugar Alternatives in Fruit Technology" (Kay O-Donald and Malcolm W. Kearsley, 2nd edition, first published 2012 by John Wiley & Sons, Ltd., Chapter 9.2.3 Lo han gou (mogroside), pages 197-199), which is herein incorporated by specific reference.

The term "product of monk fruit" as used according to the present invention means any product, preferably an extract, a juice or a puree, which may be obtained or prepared from monk fruit (luo han gou fruit). Useful products of monk fruits may be prepared by a process as disclosed in US 5,411,755, the content of which is herein incorporated by specific reference.

The term "table sugar" as used according to the present invention preferably defines sucrose.

The invention is in particular featured by the following advantages:
- The sweetener composition of the present invention enters into a caramelization process when being heated up, in particular while baking or cooking. In that regard, it surprisingly turned out that caramelization ability of a sweetener composition can be installed by using at least one reducing monosaccharide and/or at least one reducing disaccharide together with at least one amino acid. Thus while heating, in particular while baking or cooking, the sweetener composition of the present invention does not burn but caramelizes and develops a sweet caramel flavor.
- Further, the at least one sugar alcohol of the sweetener composition acts as a carrier or bulking agent. Thus, advantageously, a texture or volume of the sweetener composition similar to sucrose or other sugars may be accomplished.
- Further, the monk fruit and/or the product of monk fruit and/or the at least one sweetener of the monk fruit may be advantageously at least 300 times sweeter than sucrose (table sugar). For example, pure mogroside V, which embodies a sweetener of monk fruit, can be up to 400 times sweeter than sucrose. Thus, only a low amount of monk fruit and/or of a product of monk fruit and/or of at least one sweetener of monk fruit is necessary.
- Further, the amount of monk fruit and/or of a product of monk fruit and/or of at least one sweetener of monk fruit may be additionally reduced due to the presence of the at least one sugar alcohol in the sweetener composition.

In an embodiment, the at least one sugar alcohol is selected from the group consisting of erythritol, isomalt, lactitol, maltitol, sorbitol, mannitol, xylitol and mixtures of at least two of the afore-said sugar alcohols.

The at least one sugar alcohol may have a proportion of 60 % by weight to 95% by weight, in particular 75% by weight to 95% by weight, preferably 85% by weight to 95% by weight, related to the total weight of the sweetener composition.

Preferably, the at least one sugar alcohol is erythritol. Erythritol is a calorie-free sugar alcohol, which advantageously has no noteworthy influence on the glycemic index, and thus exercises no appreciable effect on the insulin mirror. Further, erythritol has only a little laxative effect. In particular, it may be preferred within the scope of the present invention that erythritol is the only sugar alcohol of the sweetener composition according to the present invention.

In a further embodiment, the at least one sugar alcohol is erythritol and at least one further sugar alcohol. In other words, it may be preferred within the scope of the present invention that the at least one sugar alcohol is a combination or mixture of erythritol and at least one further sugar alcohol. The at least one further sugar alcohol may be advantageously a sugar alcohol having a negative enthalpy of solution or only a low positive enthalpy of solution in water or an aqueous medium like saliva, and thus exercising no or only a minimal cooling effect in the mouth, in particular on the tongue, when being dissolved in saliva. Thus, a cooling effect of erythritol on the tongue when being dissolved in saliva may be advantageously balanced. The at least one further sugar alcohol may be selected from the group consisting of isomalt, lactitol, maltitol, sorbitol, mannitol, xylitol and mixtures of at least two of the afore-said sugar alcohols.

Preferably, the at least one sugar alcohol is erythritol and isomalt, i.e. a combination or mixture of erythritol and isomalt. Isomalt advantageously exhibits a minimal cooling effect on the tongue, lower than other sugar alcohols. Thus, isomalt is especially useful for reducing a cooling effect of erythritol on the tongue. In particular, it may be preferred within the scope of the present invention that erythritol and isomalt are the only sugar alcohols of the sweetener composition according to the present invention.

In a further embodiment, erythritol has a proportion of 15% by weight to 30% by weight, in particular 20% by weight to 30% by weight, preferably 25% by weight to 30% by weight, related to the total weight of the sweetener composition.

In a further embodiment, isomalt has a proportion of 45% by weight to 65% by weight, in particular 55% by weight to 65% by weight, preferably 60% by weight to 65% by weight, related to the total weight of the composition.

In a further embodiment, the at least one reducing monosaccharide is selected from the group consisting of fructose, glucose, galactose, mannose, ribose, arabinose, xylose, D-tagatose and mixtures of at least two of the afore-said reducing monosaccharides.

Preferably, the at least one reducing monosaccharide is fructose. Fructose advantageously allows caramelization of the sweetener composition already at lower temperatures, in particular at a temperature of 90°C to 120°C, preferably 100°C to 120°C.

Further, it may be within the scope of the present invention that the at least one reducing monosaccharide is not glucose. Thus, the influence of the sweetener composition on the insulin mirror may be advantageously reduced.

In a further embodiment, the at least one reducing monosaccharide has a proportion of 1 % by weight to 3 % by weight, in particular 2 % by weight to 3 % by weight, preferably 2,5 % by weight to 3 % by weight, related to the total weight of the composition. Thus, caramelization of the sweetener composition can be advantageously achieved based on low monosaccharide amounts, and concurrently without an appreciate impact on the insulin mirror.

In a further embodiment, the at least one reducing disaccharide is selected from the group consisting of lactose, maltose and a mixture thereof.

In a further embodiment, the at least one reducing disaccharide has a proportion of 1 % by weight to 3 % by weight, in particular 2 % by weight to 3 % by weight, preferably 2,5 % by weight to 3 % by weight, related to the total weight of the composition. Thus, caramelization of the sweetener composition can be advantageously achieved based on low disaccharide amounts, and concurrently without an appreciate impact on the insulin mirror.

Further, it may be within the scope of the present invention that the at least one reducing disaccharide is not sucrose. Thus, an influence of the sweetener composition on the insulin mirror may be advantageously minimized.

Preferably, the at least one oligosaccharide according to the present invention is an oligosaccharide having a negative enthalpy of solution or only a low positive enthalpy of solution in water or an aqueous medium like saliva and/or being able to release reducing monosaccharides and/or reducing disaccharides while heating, in particular while baking or cooking. Thus a cooling effect of the at least one sugar alcohol, in particular of erythritol, in the mouth, in particular on the tongue, may be minimized and/or the caramelization ability of the sweetener composition, in particular while baking or cooking, may be enhanced.

In a further embodiment, the at least one oligosaccharide is a fructooligosaccharide, i.e. an oligosaccharide containing fructose units or sucrose units, in particular an oligosaccharide consisting of chain-terminating glucosyl units and a repetitive fructosyl unit, which are preferably linked by β(2,1) bonds. Further, the fructooligosaccharide may have a degree of polymerization (DP) from 2 to 10. Fructooligosaccharides are especially advantageous, since they are able to minimize or balance a cooling effect of the at least one sugar, in particular of erythritol, in the mouth, in particular on the tongue and to act as caramelization boosters, without exercising a noteworthy influence on the insulin mirror.

In a further embodiment, the at least one oligosaccharide is a heterogeneous mixture of fructooligosaccharides, in particular having a degree of polymerization (DP) from 2 to 10. With respect to the advantages of such a mixture, reference is made to the previous description. The advantages already mentioned in terms of fructooligosaccharides are appropriately valid in terms of a heterogeneous mixture as disclosed in this paragraph.

Alternatively or in combination, the at least one oligosaccharide may be an oligosaccharide containing glucose units. Also a glucose unit containing oligosaccharide may be able to reduce a cooling effect of the at least one sugar alcohol, in particular of erythritol, and/or may boost the caramelization ability of the sweetener composition. The glucose unit containing oligosaccharide may have a degree of polymerization (DP) from 2 to 10, in particular 3 to 10.

In a further embodiment, the at least one oligosaccharide is a heterogeneous mixture of glucose unit containing oligosaccharides, in particular having a degree of polymerization (DP) from 2 to 10, in particular 3 to 10. With respect to the advantages of such a mixture, reference is made to the previous paragraph. The advantages already mentioned in terms of the glucose unit containing oligosaccharide are appropriately valid in terms of a heterogeneous mixture as disclosed in this paragraph.

Preferably, the at least one polysaccharide according to the present invention is a polysaccharide having a negative enthalpy of solution or only a low positive enthalpy of solution in water or an aqueous medium like saliva and/or being able to release reducing monosaccharides and/or reducing disaccharides while heating, in particular while baking or cooking. Thus a cooling effect of the at least one sugar alcohol, in particular of erythritol, in the mouth, in particular on the tongue, may be minimized and/or the caramelization ability of the sweetener composition, in particular while baking or cooking, may be enhanced.

In a further embodiment, the at least one polysaccharide is a fructopolysaccharide, i.e. a polysaccharide containing fructose units or sucrose units. Preferably, the fructopolysaccharide has a degree of polymerization (DP) from 11 to 60. Also Fructopolysaccharides are especially advantageous, since they are also able to minimize or balance a cooling effect of the at least one sugar, in particular of erythritol, in the mouth, in particular on the tongue and to act as caramelization boosters, without exercising a noteworthy influence on the insulin mirror.

In a further embodiment, the at least one polysaccharide is a heterogeneous mixture of fructopolysaccharides, in particular having a degree of polymerization (DP) from 11 to 60. With respect to the advantages of such a mixture, reference is made to the previous paragraph. The advantages already mentioned in terms of fructopolysaccharides are appropriately valid in terms of a heterogeneous mixture as disclosed in this paragraph.

In a further embodiment, the at least one oligosaccharide and/or the at least one polysaccharide is/are inulin. Inulin is a heterogeneous mixture of fructose polymers. It consists of chain-terminating glucosyl units and a repetitive fructosyl unit, which are linked by β(2,1) bonds. The degree of polymerization (DP) of the inulin may range from 2 to 60. With respect to the advantages of inulin, reference is made to the previous description. The advantages already mentioned in terms of fructooligosaccharides and/or fructopolysaccharides are appropriately valid in terms of inulin.

Alternatively or in combination, the at least one polyaccharide may be a polysaccharide containing glucose units. Also a glucose unit containing polysaccharide may be able to reduce a cooling effect of the at least one sugar alcohol, in particular of erythritol, and/or may boost the caramelization ability of the sweetener composition.

In a further embodiment, the at least one polysaccharide is a heterogeneous mixture of glucose unit containing polysaccharides, in particular having a degree of polymerization (DP) from 11 to 17. With respect to the advantages of such a mixture, reference is made to the previous paragraph. The advantages already mentioned in terms of the glucose unit containing polysaccharide are appropriately valid in terms of a heterogeneous mixture as disclosed in this paragraph.

In a further embodiment, the at least one oligosaccharide and/or the at least one polysaccharide is/are maltodextrin. Maltodextrin consists of D-glucose units connected in chains of variable length. The glucose units are primarily linked with α(1,4) glycosidic bonds. Maltodextrin is typically composed of a mixture of chains that vary from 3 to 17 glucose units long. With respect to the advantages of such a mixture, reference is made to the previous description. The advantages already mentioned in terms of the glucose unit containing oligosaccharide and/or the glucose unit containing polysaccharide are appropriately valid in terms of a heterogeneous mixture as disclosed in this paragraph.

In a further embodiment, the at least one oligosaccharide and/or the at least one polysaccharide, in particular inulin and/or the at least one fructooligosaccharide, has a proportion of 1 % by weight to 3 % by weight, in particular 2 % by weight to 3 % by weight, preferably 2,5 % by weight to 3 % by weight, related to the total weight of the composition.

In a further embodiment, the at least one amino acid is selected from the group consisting of glycine, lysine, asparagine, aspartic acid, arginine, histidine, glutamine, glutamic acid, tryptophan, tyrosine, phenyl alanine, alanine, β-alanine, cysteine, methionine, proline, threonine, serine, carnosine and mixtures of at least two of the afore-said amino acids.

Preferably, the at least one amino acid is glycine and/or lysine. In particular, it may be preferred within the scope of the present invention that glycine and/or lysine is/are the only amino acid/amino acids of the sweetener composition of the present invention.

More preferably, the at least one amino acid is glycine. In particular, it may be preferred within the scope of the present invention that glycine is the only amino acid of the sweetener composition of the present invention. Glycine has the additional advantage that it may act as a sweetener and thus, along the monk fruit and/or the product of monk fruit and/or the at least one sweetener of monk fruit, is able to replace the sweetness of table sugar or other sugars like glucose.

In a further embodiment, the at least one amino acid has a proportion of 0,7 % by weight to 4,5 % by weight, in particular 1,4 % by weight to 2,7 % by weight, preferably 2,0 % by weight to 3,0 % by weight, related to the total weight of the composition.

In a further embodiment, glycine has a proportion of 0,2 % by weight to 1,5 % by weight, in particular 0,4 % by weight to 1,2 % by weight, preferably 0,5 % by weight to 1,0 % by weight, related to the total weight of the composition.

In a further embodiment, lysine has a proportion of 0,5 % by weight to 3 % by weight, in particular 1,0 % by weight to 2,5 % by weight, preferably 1,5 % by weight to 2 % by weight, related to the total weight of the composition.

Preferably, the at least one anti-laxative agent according to the present invention has a negative enthalpy of solution or only a low positive enthalpy of solution in water or an aqueous medium like saliva. Thus, the at least one anti-laxative agent is (also) advantageously adapted to minimize a cooling effect of the at least one sugar alcohol, in particular of erythritol.

The at least one anti-laxative agent may be a thickening agent, in particular a gelling agent. Thus, the at least one laxative agent is advantageously able to absorb or bind water which is released into the intestinal lumen and otherwise would result in a laxative effect.

Preferably, the at least one laxative agent is selected from the group consisting of alginic acid, salts of alginic acid, methylcellulose, sodium carboxymethylcellulose, cross-linked sodium carboxymethylcellulose, carboxymethylcellulose, ethylmethylcellulose, hydroxypropylcellulose, methylhydroxypropylcellulose, methylcellulose, cassia rubber, soybean hemicellulose, konjac-rubber, gellan or gellan gum, Tara gum, karaya or karaya gum, xanthan or xanthan gum, rubber arabic, gum tragacanth, guar gum, locust bean flour, carrageen, agar-agar, tannins and mixtures of at least two of the afore-said anti-laxative agents.

The salts of alginic acid may be selected from the group consisting of sodium alginate, potassium alginate, ammonium alginate, calcium alginate and mixtures of at least two of the afore-said salts of alginic acid.

The anti-laxative effect of the above-mentioned tannins is advantageously due to their contracting effect on the villi which leads to a reduction of the intestinal lumen's surface. Thus, the amount of water which may be released into the intestinal lumen may be advantageously reduced.

Preferably, the at least one anti-laxative agent is an alginate, in particular selected from the group consisting of sodium alginate, potassium alginate, ammonium alginate, calcium alginate and mixtures of at least two of the afore-said alginates, and/or methylcellulose. In particular, it may be within the scope of the present invention that alginate, in particular selected from the group consisting of sodium alginate, potassium alginate, ammonium alginate, calcium alginate and mixtures of at least two of the afore-said alginates, and/or methylcellulose is/are the only anti-laxative agent/agents of the sweetener composition of the present invention.

In a further embodiment, the at least one anti-laxative agent has a proportion of 0,3 % by weight to 1,8 % by weight, in particular 0,4 % by weight to 1,6 % by weight, preferably 0,8 % by weight to 1,2 % by weight, related to the total weight of the composition.

In a further embodiment, alginate, preferably sodium alginate, has a proportion of 0,2 % by weight to 0,8 % by weight, in particular 0,3 % by weight to 0,8 % by weight, preferably 0,4 % by weight to 0,6 % by weight, related to the total weight of the composition.

In a further embodiment, methylcellulose has a proportion of 0,1 % by weight to 1,0 % by weight, in particular 0,2 % by weight to 0,8 % by weight, preferably 0,4 % by weight to 0,6 %, related to the total weight of the composition.

In a further embodiment, the at least one biocompatible alkali metal salt is selected from the group consisting of alkali metal citric acid salt, alkali metal phosphoric acid salt and mixtures thereof. Both, alkali metal citric acid salts and alkali metal phosphoric acid salts have the advantage that they are able to minimize a cooling effect of the at least one sugar alcohol. This is especially advantageous where the at least one sugar alcohol comprises or is erythritol. The use of an alkali metal citric acid salt has the additional advantage that it is able to increase an aqueous pH-value, thereby improving a gelling effect, and thus a water absorbing or binding effect, of the at least one anti-laxative agent.

Preferably, the alkali metal citric acid salt is selected from the group consisting of monosodium citrate (NaC₆H₇O₇), disodium citrate (Na₂C₆H₆O₇), trisodium citrate (Na₃C₆H₅O₇), monopotassium citrate (KC₆H₇O₇), dipotassium citrate (K₂C₆H₆O₇), tripotassium citrate (K₃C₆H₅O₇) and mixtures of at least two of the afore-said alkali metal citric acid salts.

The alkali metal phosphoric acid salt is preferably selected from the group consisting of monosodium dihydrogen phosphate, disodium hydrogen phosphate, trisodium phosphate, monopotassium dihydrogen phosphate, dipotassium hydrogen phosphate, tripotassium phosphate and mixtures of at least two of the afore-said alkali metal phosphoric acid salts.

More preferably, the at least one biocompatible alkali metal salt is selected from the group consisting of monosodium citrate (NaC₆H₇O₇), disodium citrate (Na₂C₆H₆O₇), trisodium citrate (Na₃C₆H₅O₇), monopotassium citrate (KC₆H₇O₇), dipotassium citrate (K₂C₆H₆O₇), tripotassium citrate (K₃C₆H₅O₇), monosodium dihydrogen phosphate, disodium hydrogen phosphate, trisodium phosphate, monopotassium dihydrogen phosphate, dipotassium hydrogen phosphate, tripotassium phosphate and mixtures of at least two of the afore-said alkali metal salts.

In particular, the at least one biocompatible alkali metal salt may be trisodium citrate and/or dipotassium hydrogen phosphate.

In a further embodiment, the at least one biocompatible alkali metal salt has a proportion of 0,2 % by weight to 1,5 % by weight, in particular 0,4 % by weight to 1,2 % by weight, preferably 0,6 % by weight to 0,9 % by weight, related to the total weight of the composition.

In a further embodiment, the alkali metal citric acid salt, in particular trisodium citrate, has a proportion of 0,1 % by weight to 0,5 % by weight, in particular 0,2 % by weight to 0,4 % by weight, preferably 0,2 % by weight to 0,3 % by weight, related to the total weight of the composition.

In a further embodiment, the alkali metal phosphoric acid salt, in particular dipotassium hydrogen phosphate, has a proportion of 0,1 % by weight to 1,0 % by weight, in particular 0,2 % by weight to 0,8 % by weight, preferably 0,4 % by weight to 0,6 % by weight, related to the total weight of the composition.

In a further embodiment, the at least one biocompatible alkaline earth metal salt is selected from the group consisting of alkaline earth metal citric acid salt, alkaline earth metal phosphoric acid salt and mixtures thereof. Both, alkaline earth metal citric acid salts and alkaline earth metal phosphoric acid salts are advantageously adapted to reduce a cooling effect of the at least one sugar alcohol on the tongue. This is especially advantageous where the at least one sugar alcohol comprises or is erythritol. Alkaline earth metal citric acid salts have the additional advantage that they are able to increase the pH-value, thereby improving a gelling effect of the at least one anti-laxative agent.

Preferably, the alkaline earth metal citric acid salt is selected from the group consisting of monocalcium citrate, dicalcium citrate, tricalcium citrate and mixtures of at least two of the afore-said alkaline earth metal citric acid salts.

The alkaline earth metal phosphoric acid salt is preferably selected from the group consisting of calcium phosphate, magnesium phosphate and mixtures of the afore-said alkaline earth metal phosphoric acid salts.

More preferably, the at least one biocompatible alkaline earth metal salt is selected from the group consisting of monocalcium citrate, dicalcium citrate, tricalcium citrate, calcium phosphate, magnesium phosphate and mixtures of at least two of the afore-said alkaline earth metal salts.

In a further embodiment, the product of monk fruit is selected from the group consisting of juice, puree, and extract. The juice may be a diluted juice, a concentrated juice or a dried juice. The extract may be a liquid extract or a solid extract, in particular a dried and/or powdery extract.

In a further embodiment, the product of monk fruit is a composition, in particular in the form of a juice, a puree or an extract. The juice may be a diluted juice, a concentrated juice or a dried juice. The extract may be a liquid extract or a solid extract, in particular a dried and/or powdery extract.

Preferably, the product of monk fruit is a juice or juice composition, The juice or juice composition may comprise, in particular on a dry basis, 0,1 % by weight to 15 % by weight of mogrosides and/or siamenoside, in particular mogroside IV, mogroside V, 11-oxo-mogroside V, siamenoside I or mixtures thereof. In particular, the juice may be prepared as disclosed in US 5,411,755 whose content, in particular concerning the preparation and/or the composition of the juice, is incorporated herein by specific reference.

In a further embodiment, the product of monk fruit comprises at least one sweetener, in particular at least one sweet terpene glycoside.

Preferably, the at least one sweetener, in particular the at least one sweet terpene glycoside, is selected from the group consisting of at least one mogroside, at least one siamenoside, at least one neomogroside and mixtures of at least two of the afore-said sweeteners, in particular sweet terpene glycosides.

The at least one mogroside may be selected from the group consisting of mogroside I, mogroside II mogroside III mogroside IV, mogroside V, and mixtures of at least two of the afore-said mogrosides. V. In particular, the at least one mogroside may be mogroside IV and/or mogroside V, preferably mogroside V.

More preferably, the at least one mogrosides is selected from the group consisting of mogroside I, mogroside II A1, mogroside II B, 7-oxomogroside II E, 11-oxomogroside A1, mogroside III A2, 11-deoxymogroside III, 11-oxomogroside IV A, mogroside V, 7-oxomogroside V, 11-oxomogroside V, mogroside VI and mixtures of at least two of the afore-said mogrosides.

The at least one siamenoside may be siamenoside I.

Preferably, the product of monk fruit comprises at least one sweetener, in particular at least one sweet terpene glycoside, which is selected from the group consisting of mogroside I, mogroside II A1, mogroside II B, 7-oxomogroside II E, 11-oxomogroside A1, mogroside III A2, 11-deoxymogroside III, 11-oxomogroside IV A, mogroside V, 7-oxomogroside V, 11-oxomogroside V, mogroside VI, siamenoside I and mixtures of at least two of the afore-said sweet terpene glycosides.

In a further embodiment, the at least one sweetener of monk fruit is at least one sweet terpene glycoside. Preferably, the at least one sweet terpene glycoside is selected from the group consisting of at least one mogroside, at least one siamenoside and mixtures of at least two of the afore-said sweet terpene glycosides.

The at least one mogroside may be selected from the group consisting of mogroside I, mogroside II mogroside III mogroside IV, mogroside V and mixtures of at least two of the afore-said mogrosides. In particular, the mogroside may be mogroside IV and/or mogroside V, preferably mogroside V.

More preferably, the at least one mogroside is selected from the group consisting of mogroside I, mogroside II A1, mogroside II B, 7-oxomogroside II E, 11-oxomogroside A1, mogroside III A2, 11-deoxymogroside III, 11-oxomogroside IV A, mogroside V, 7-oxomogroside V, 11-oxomogroside V, mogroside VI and mixtures of at least two of the afore-said mogrosides.

The at least one siamenoside may be siamenoside I.

Preferably, the at least one sweetener of monk fruit is selected from the group consisting of mogroside I, mogroside II A1, mogroside II B, 7-oxomogroside II E, 11-oxomogroside A1, mogroside III A2, 11-deoxymogroside III, 11-oxomogroside IV A, mogroside V, 7-oxomogroside V, 11-oxomogroside V, mogroside VI, siamenoside I and mixtures of at least two of the afore-mentioned sweeteners.

In a further embodiment, the monk fruit and/or the product of monk fruit and/or the at least one sweetener of monk fruit, have/has a proportion of 0,1 % by weight to 1,0 % by weight, in particular 0,2 % by weight to 0,8 % by weight, preferably 0,4 % by weight to 0,6 % by weight, related to the total weight of the composition.

In a further embodiment, which may also embody a further independent aspect of the present invention, the sweetener composition comprises or includes the following ingredients:
- erythritol and/or isomalt,
- fructose,
- inulin and/or a fructooligosaccharide,
- glycine and/or lysine, preferably glycine,
- at least one anti-laxative agent, preferably sodium alginate and/or methylcellulose,
- at least one alkali metal salt, preferably trisodium citrate, and dipotassium hydrogen phosphate, and/or at least one alkaline earth metal salt and
- monk fruit and/or a product of monk fruit and/or at least one sweetener of monk fruit.

With respect to further features and advantages of the above embodiment and aspect of the invention, respectively, reference is made in its entirety to the previous description. The features and advantages disclosed in respect of the sweetener composition in the previous description do accordingly apply with respect to the sweetener composition as defined in the above embodiment.

In a further embodiment, the sweetener composition according to the present invention comprises or includes the following ingredients to 100 % by weight:
- 15 % by weight to 30 % by weight of erythritol and/or 45 % by weight to 65 % by weight of isomalt,
- 1 % by weight to 3 % by weight of fructose,
- 1 % by weight to 3 % by weight of inulin and/or a fructooligosaccharide,
- 0,5 % by weight to 3 % by weight of lysine and/or 0,2 % by weight to 1,5 % by weight of glycine,
- 0,2 % by weight to 0,8 % by weight of alginate, in particular sodium alginate, and/or 0,1 % by weight to 1,0 % by weight of methylcellulose,
- 0,1 % by weight to 0,5 % by weight of at least one alkali metal citric acid salt, preferably trisodium citrate, and/or 0,1 % by weight to 1,0 % by weight of at least one alkali metal phosphoric acid salt, in particular dipotassium hydrogen phosphate, and
- 0,1 % by weight to 1,0 % by weight of monk fruit and/or of the product of monk fruit and/or of the at least one sweetener of monk fruit.

With respect to further features and advantages of the above embodiment, reference is made in its entirety to the previous description. The features and advantages disclosed in respect of the sweetener composition in the previous description do accordingly apply with respect to the sweetener composition as defined in the above embodiment.

In a further embodiment, the sweetener composition may consist of the following ingredients:
- at least one sugar alcohol,
- at least one reducing monosaccharide and/or at least one reducing disaccharide,
- at least one oligosaccharide and/or at least one polysaccharide,
- at least one amino acid,
- at least one anti-laxative agent,
- at least one biocompatible alkali metal salt and/or at least one biocompatible alkaline earth metal salt and
- monk fruit and/or a product of monk fruit and/or at least one sweetener of monk fruit.

With respect to further features and advantages, reference is made in its entirety to the previous description. The features and advantages described with respect to the sweetener composition in the previous description do accordingly apply with respect to the sweetener composition as defined in the above embodiment.

In particular, the sweetener composition may consist of the following ingredients:
- erythritol and/or isomalt,
- fructose,
- inulin and/or a fructooligosaccharide,
- glycine and/or lysine, preferably glycine,
- at least one anti-laxative agent, preferably sodium alginate and/or methylcellulose,
- at least one alkali metal salt, preferably trisodium citrate, and dipotassium hydrogen phosphate, and/or at least one alkaline earth metal salt and
- monk fruit and/or a product of monk fruit and/or at least one sweetener of monk fruit.

With respect to further features and advantages, reference is made in its entirety to the previous description. The features and advantages described with respect to the sweetener composition in the previous description do accordingly apply with respect to the sweetener composition as defined in the above embodiment.

In a further embodiment, the sweetener composition according to the present invention consists of the following ingredients to 100 % by weight:
- 15 % by weight to 30 % by weight of erythritol and/or 45 % by weight to 65 % by weight of isomalt,
- 1 % by weight to 3 % by weight of fructose,
- 1 % by weight to 3 % by weight of inulin and/or a fructooligosaccharide,
- 0,5 % by weight to 3 % by weight of lysine and/or 0,2 % by weight to 1,5 % by weight of glycine,
- 0,2 % by weight to 0,8 % by weight of alginate, in particular sodium alginate, and/or 0,1 % by weight to 1,0 % by weight of methylcellulose,
- 0,1 % by weight to 0,5 % by weight of at least one alkali metal citric acid salt, in preferably trisodium citrate, and/or 0,1 % by weight to 1,0 % by weight of at least one alkali metal phosphoric acid salt, in particular dipotassium hydrogen phosphate, and
- 0,1 % by weight to 1,0 % by weight of monk fruit and/or of the product of monk fruit and/or of the at least one sweetener of monk fruit.

With respect to further features and advantages of the above embodiment, reference is made in its entirety to the previous description. The features and advantages disclosed in respect of the sweetener composition in the previous description do accordingly apply with respect to the sweetener composition as defined in the above embodiment.

In a further embodiment, the sweetener composition is a solid, in particular crystalline, composition. Preferably, the sweetener composition is a crystalline powder or crystalline granules.

In a further embodiment, the sweetener composition is an uncolored composition. In particular, the sweetener composition may be an uncolored solid composition, in particular an uncolored crystalline composition. For example, the sweetener composition may be an uncolored crystalline powder or uncolored crystalline granules.

According to a further aspect, the invention refers to a consumable, in particular edible consumable, comprising a sweetener composition of the present invention.

Principally, the consumable may be a food product, a beverage product, a nutraceutical product, a pharmaceutical product, a sports product, a tobacco product or a cosmetic product.

Preferably, the consumable according to the present invention is a food product or a beverage product.

The food product may be selected from the group consisting of bakery product (pastries), confectionary product, dessert product, cereal product, frozen diary product, meat, diary product, condiment, snack bar, soup, dressing, prepared food, baby food, diet preparation, syrup, food coating, dried food, sauce, gravy, jam/jelly, spread, butter, breading, spice mix, frosting and coating.

The beverage product may be selected from the group consisting of concentrated beverage mix, carbonated beverage, non-carbonated beverage, fruit-flavored beverage, fruit juice, tea, milk, coffee, nectar, powdered soft drink, liquid concentrate, milk drink, smoothie, alcoholic beverage, flavored water and mixtures of at least two of the afore-said beverage products.

Preferably, the consumable according to the present invention is a bakery product.

Further, it may be preferred that the consumable is a caramel coloring composition, preferably for coloring beverages, in particular non-alcoholic beverages like coca cola.

With respect to further features and advantages, reference is made in its entirety to the previous description. The features and advantages described with respect to the sweetener composition during the previous description do accordingly apply with respect to the consumable.

Finally, the present invention relates to the use of a sweetener composition as disclosed according to the present invention for substitution of sugar, preferably for substitution of sucrose (table sugar) and/or for substitution of glucose, in particular for substitution of uncolored sucrose (uncolored table sugar) and/or for substitution of uncolored glucose.

With respect to further features and advantages, reference is made in its entirety to the previous description. The features and advantages described with respect to the sweetener composition in the previous description do accordingly apply with respect to the use of such a composition for substituting a sugar, in particular sucrose and/or glucose.

Further features and advantages of the invention are given in the following description of a preferred embodiment in the form of an example. The example serves only to explain the present invention, which is by no means limited to the example.

### EXAMPLE SECTION

### 1. Ingredients, materials and apparatus for manufacture of a sweetener composition:

The following ingredients materials and apparatus were used for manufacturing a sweetener composition according to the present invention:

### 1.1 Ingredients for the sweetener composition

**Table 1: ingredients (in g and in % by weight) for the sweetener composition**

| ***Ingredients*** | ***grams (g) of the ingredients to produce 100g of the Sweetener composition*** | ***percentages of weight of the ingredients in the final sweetener composition*** |
|---|---|---|
| Erythritol | 26,966 g | 26,966% |
| Isomalt | 62,921 g | 62,921% |
| Fructose | 2,697 g | 2,697% |
| Inulin / FOS | 2,697 g | 2,697% |
| Lysine | 1,798 g | 1,798% |
| Glycine | 0,899 g | 0,899% |
| Algin (Sodium Alginate) | 0,449 g | 0,449% |
| Methlycellulose | 0,449 g | 0,449% |
| Sodium citrate | 0,225g | 0,225% |
| Dipotassium phosphate | 0,449g | 0,449% |
| Monk fruit | 0,449g | 0,449% |

### 1.2 Material:

Distilled Water: 200g

### 1.3 Apparatus:

- Glass Beaker
- Magnetic stirrer
- Magnetic bar for the stirrer
- Scale
- Spoon
- Hotplate
- Thermometer
- Flat metal bowl
- Freezer (lower than 30°C)
- Vacuumdryer (Endvacuum lower than 0,04 mbar)

### 2. Manufacture of the sweetener composition

All ingredients except of alginate and methylcellulose were added into a Glass Beaker with magnetic bar using the amounts as specified in table 1. Then 200g of warm distilled water was added and the mixture was stirred. To solute everything evenly the mixture was brought to a temperature of up to 55°C. A magnetic stirrer with an integrated hotplate was found to be the best solution to have full control of the creation of an oversaturated solution. When the solution reached the temperature of 55 °C, the bar was taken from the hotplate and sodium alginate and methylcellulose were added using the amounts as specified in table 1. In the moment the sodium alginate and methylcellulose were fully dissolved in the solution, the mixture was ready to be poured into a flat metal bowl to ensure a quick cooling down of the solution and a faster drying process. After the solution was poured into the bowl it was put into a freezer, where it was cooled down to -30° C. Afterwards the bowl with the frozen solution was put out of the freezer and put into a vacuum dryer with an endvacuum of at lower than 0,04 mbar to get rid of the last remaining water and to get to the final crystalline granulate of the sweetener composition.

### 3. Process of producing a dry caramel based on the sweetener composition as manufactured by the process as detailed under 2.

### Ingredients:

Sweetener composition as manufactured according to 2.

### Material:

- Direct and indirect heating
- Metal pot
- Spoon

### Procedure:

A metal pot was put on a hot plate or oven. The sweetener composition as manufactured according to 2. was poured into the metal pot. Then, the direct or indirect heat source was turned on. When the sweetener composition started to melt at 90°C and the first color changes were observed, stirring was started. The whole mixture was melted at a temperature level at 140°C, where it also got to his full caramel flavor. Above this temperature level, the caramel developed a very dark color and more and more burned flavors, which are often perceived as off flavors in most food applications.

## Claims

1. Sweetener composition comprising the following ingredients:
- at least one sugar alcohol,
- at least one reducing monosaccharide and/or at least one reducing disaccharide,
- at least one oligosaccharide and/or at least one polysaccharide,
- at least one amino acid,
- at least one anti-laxative agent,
- at least one biocompatible alkali metal salt and/or at least one biocompatible alkaline earth metal salt and
- monk fruit and/or a product of monk fruit and/or at least one sweetener of monk fruit.

2. Sweetener composition according to claim 1, wherein the at least one sugar alcohol is erythritol or a combination of erythritol and at least one further sugar alcohol, preferably isomalt.

3. Sweetener composition according to claim 2, wherein erythritol has a proportion of 15 % by weight to 30 % by weight, in particular 20 % by weight to 30 % by weight, preferably 25 % by weight to 30 % by weight, related to the total weight of the composition and/or wherein isomalt has a proportion of 45 % by weight to 65 % by weight, in particular 55 % by weight to 65 % by weight, preferably 60 % by weight to 65 % by weight, related to the total weight of the composition.

4. Sweetener composition according to any of the preceding claims, wherein the at least one reducing monosaccharide is selected from the group consisting of fructose, glucose, galactose, mannose, ribose, arabinose, xylose, D-tagatose and mixtures of at least two of the afore-said reducing monosaccharides and/or wherein the at least one reducing disaccharide is selected from the group consisting of lactose, maltose or a mixture thereof.

5. Sweetener composition according to any of the preceding claims, wherein the at least one reducing monoaccharide and/or the at least one reducing disaccharide has a proportion of 1 % by weight to 3 % by weight, in particular 2 % by weight to 3 % by weight, preferably 2,5 % by weight to 3 % by weight, related to the total weight of the composition.

6. Sweetener composition according to any of the preceding claims, wherein the at least one oligosaccharide is a fructooligosaccharide and/or the at least one polysaccharide is a fructopolysaccharide, in particular wherein the at least one oligosaccharide and/or the at least one polysaccharide is inulin.

7. Sweetener composition according to any of the preceding claims, wherein the at least one amino acid is selected from the group consisting of glycine, lysine, asparagine, aspartic acid, arginine, histidine, glutamine, glutamic acid, tryptophan, tyrosine, phenyl alanine, alanine, β-alanine, cysteine, methionine, proline, threonine, serine, carnosine and mixtures of at least two of the afore-said amino acids.

8. Sweetener composition according to any of the preceding claims, wherein the at least one amino acid is glycine and/or lysine, preferably glycine, in particular wherein glycine has a proportion of 0,2 % by weight to 1,5 % by weight, in particular 0,4 % by weight to 1,2 % by weight, preferably 0,5 % by weight to 1,0 % by weight, related to the total weight of the composition, and/or wherein lysine has a proportion of 0,5 % by weight to 3 % by weight, in particular 1,0 % by weight to 2,5 % by weight, preferably 1,5 % by weight to 2 % by weight, related to the total weight of the composition.

9. Sweetener composition according to any of the preceding claims, wherein the at least one anti-laxative agent is selected from the group consisting alginic acid, salts of alginic acid, methylcellulose, sodium carboxymethylcellulose, cross-linked sodium carboxymethylcellulose, carboxymethylcellulose, ethylmethylcellulose, hydroxypropylcellulose, methylhydroxypropylcellulose, methylcellulose, cassia rubber, soybean hemicellulose, konjac-rubber, gellan or gellan gum, Tara gum, karaya or karaya gum, xanthan or xanthan gum, rubber arabic, gum tragacanth, guar gum, locust bean flour, carrageen, agar-agar, tannins and mixtures of at least two of the afore-said anti-laxative agents.

10. Sweetener composition according to any of the preceding claims, wherein the at least one anti-laxative agent is an alginate, preferably sodium alginate, and/or methylcellulose, in particular wherein alginate, preferably sodium alginate, has a proportion of 0,2 % by weight to 0,8 % by weight, in particular 0,3 % by weight to 0,8 % by weight, preferably 0,4 % by weight to 0,6 % by weight, related to the total weight of the composition, and/or wherein methylcellulose has a proportion of 0,1 % by weight to 1,0 % by weight, in particular 0,2 % by weight to 0,8 % by weight, preferably 0,4 % by weight to 0,6 % by weight, related to the total weight of the composition.

11. Sweetener composition according to any of the preceding claims, wherein the at least one biocompatible alkali metal salt is selected from the group consisting of alkali metal citric acid salts, alkali metal phosphoric acid salts, and mixtures thereof, and/or wherein the at least one biocompatible alkaline earth metal salt is selected from the group consisting of alkaline earth metal citric acid salts, alkaline earth metal phosphoric acid salts and mixtures thereof, in particular wherein the at least one biocompatible alkali metal salt is selected from the group consisting of monosodium citrate, disodium citrate, trisodium citrate, monopotassium citrate, dipotassium citrate, tripotassium citrate, monosodium dihydrogen phosphate, disodium hydrogen phosphate, trisodium phosphate, monopotassium dihydrogen phosphate, dipotassium hydrogen phosphate, tripotassium phosphate and mixtures of at least two of the afore-said alkali metal salts, and/or wherein the at least one biocompatible alkaline earth metal salt is selected from the group consisting of monocalcium citrate, dicalcium citrate, tricalcium citrate, calcium phosphate, magnesium phosphate and mixtures of at least two of the afore-said alkaline earth metal salts.

12. Sweetener composition according to any of the preceding claims, wherein the product of monk fruit is a composition, in particular in the form of a juice, a puree or an extract, and/or wherein the product of monk fruit comprises at least one sweet terpene glycoside, in particular selected from the group consisting of mogroside I, mogroside II A1, mogroside II B, 7-oxomogroside II E, 11-oxomogroside A1, mogroside III A2, 11-deoxymogroside III, 11-oxomogroside IV A, mogroside V, 7-oxomogroside V, 11-oxomogroside V, mogroside VI, siamenoside I and mixtures of at least two of the afore-mentioned sweet terpene glycosides, and/or wherein the at least one sweetener of monk fruit is selected from the group consisting of mogroside I, mogroside II A1, mogroside II B, 7-oxomogroside II E, 11-oxomogroside A1, mogroside III A2, 11-deoxymogroside III, 11-oxomogroside IV A, mogroside V, 7-oxomogroside V, 11-oxomogroside V, mogroside VI, siamenoside I and mixtures of at least two of the afore-mentioned sweet terpene glycosides.

13. Sweetener composition according to any of the preceding claims, wherein the at least one biocompatible alkali metal salt has a proportion of 0,2 % by weight to 1,5 % by weight, in particular 0,4 % by weight to 1,2 % by weight, preferably 0,6 % by weight to 0,9 % by weight, related to the total weight of the composition, and/or wherein monk fruit and/or the product of monk fruit and/or the at least one sweetener of monk fruit have/has a proportion of 0,1 % by weight to 1,0 % by weight, in particular 0,2 % by weight to 0,8 % by weight, preferably 0,4 % by weight to 0,6 %, related to the total weight of the composition.

14. Consumable, in particular edible consumable, comprising a sweetener composition according to any of the preceding claims.

15. Sweetener composition according to any of the claims 1 to 13 comprising the following ingredients:
- erythritol and/or isomalt,
- fructose,
- inulin and/or a fructooligosaccharide,
- glycine and/or lysine, preferably glycine,
- at least one anti-laxative agent, preferably sodium alginate and/or methylcellulose,
- at least one alkali metal salt, preferably trisodium citrate, and dipotassium hydrogen phosphate, and/or at least one alkaline earth metal salt and
- monk fruit and/or a product of monk fruit and/or at least one sweetener of monk fruit.
